Europäisches Patentamt

European Patent Office

Office européen des brevets

(19)

(11) Publication number: **0 320 484**
**A2**

(12) **EUROPEAN PATENT APPLICATION**

(21) Application number: **88870183.6**

(22) Date of filing: **08.12.88**

(51) Int. Cl.⁴: **C 07 C 102/00**
**C 07 C 103/38**

(30) Priority: **09.12.87 US 130693**

(43) Date of publication of application:
**14.06.89 Bulletin 89/24**

(84) Designated Contracting States:
**AT BE CH DE ES FR GB GR IT LI LU NL SE**

(71) Applicant: **Monsanto Company**
**Patent Department 800 North Lindbergh Boulevard**
**St. Louis Missouri 63167-7020 (US)**

(72) Inventor: **Hoekstra, Roger John**
**1344 South Geyer Road**
**Kirkwood Missouri 63122 (US)**

**Teramura, Douglas Hideyo**
**1026K rue de la Banque**
**Creve Coeur Missouri 63141 (US)**

(74) Representative: **McLean, Peter et al**
**Monsanto Europe S.A. Patent Department Avenue de**
**Tervuren 270-272 Letter Box No 1**
**B-1150 Brussels (BE)**

(54) **Purification of N-acetyl aminophenols.**

(57) N-acetyl aminophenols contaminated with chromogenic impurities are purified via an improved process. In this process, N-acetyl aminophenol is crystallized from solvent media. The resultant solvent media phase is treated with an ion exchange resin to remove the chromogenic impurities contained therein and recycled for use as the solvent media to crystallize subsequent batches of N-acetyl aminophenol.

EP 0 320 484 A2

Bundesdruckerei Berlin

**Description**

## PURIFICATION OF N-ACETYL AMINOPHENOLS

### BACKGROUND OF THE INVENTION

#### 1. Field of the Invention

This invention relates to a process for the purification of N-acetyl aminophenols. More particularly, this invention relates to a process for the purification of N-acetyl aminophenols containing chromogenic impurities.

N-acetyl aminophenols, particularly the lower molecular weight members such as the N-acetyl alkyl-substituted aminophenols containing lower alkyl nuclear substituents, e.g., having from 1 to 4 carbon atoms, and especially N-acetyl-p-aminophenol, have found extensive use in the pharmaceutical industry as analgesics.

The class of compounds known as aminophenols, and especially ortho- and para-aminophenols, are known to oxidize readily to form quinone, quinonimine, and meriquinonimine color body impurities which impart discoloration to the aminophenol. These impurities are present in crude N-acetyl aminophenol products prepared by reacting an aminophenol with an acetylating agent such as acetic anhydride, mixtures of acetic anhydride and acetic acid, acetic acid, and the like. When such crude N-acetyl aminophenol is to be used for medicinal purposes or as intermediates in the preparation of other compounds having medicinal uses, the N-acetyl aminophenol must be purified to remove chromogenic impurities so that it is essentially color-free and retains such color-free state over prolonged periods of storage.

#### 2. Description of the Prior Art

Procedures to prepare N-acetyl aminophenols are generally well known to those skilled in the art. In general, such procedures involve acetylation of an aminophenol, such as p-aminophenol, with acetic anhydride, acetic acid, and mixtures thereof at atmospheric pressure and temperatures ranging from room temperature up to about 130°C. Variations of this general procedure are disclosed in U.S. Patents 3,042,719, 3,113,150, and 3,781,354. However, the product obtained is often unsatisfactory due to the readily oxidizable nature of aminophenols and the consequent formation of chromogenic impurities which are carried over to the acetylated product.

In an attempt to overcome the difficulties associated with the production of purified color-free N-acetyl aminophenols, various decolorization and purification methods and processes have been suggested. These include, for example, the use of boron compounds (U.S. Patents 2,945,870, 3,081,321, 3,081,322, and 4,264,526); reducing agents, such as sulfides, sulfites, and formamidinesulfinic acid (U.S. Patents 2,478,114, 3,042,719, and 4,474,985); alkaline treatments (U.S. Patents 2,013,394 and 2,822,370); acid-treated carbon and a metal chelating agent (U.S. Patent 3,113,150); and iron (III) chloride (U.S. Patent 3,781,354), as well as the usual washing and recrystallization techniques. Although these various prior art methods and processes are all effective to a certain degree in obtaining an N-acetyl aminophenol which is relatively color-free, such methods and processes are either unduly complicated and costly or, more important, do not remove completely the chromogenic impurities. Treatment with reducing agents, for example, serves mainly to convert the chromogenic impurities (which may be present as colored compounds) to a colorless form which, upon oxidation with air, reverts readily to the original colored form. As a result, formulation of such an N-acetyl aminophenol into a pharmaceutical product and subsequent warehouse and retail storage of the pharmaceutical product, e.g., analgesic tablets, will result in a product which gradually degrades over time in color from the original white product. In addition, the prior art methods and processes are not readily adaptable to continuous and semicontinuous operations involving recycling of process streams to recover residual product, while simultaneously minimizing or preventing altogether contamination of the N-acetyl aminophenol product with chromogenic impurities or contaminants contained in such recycled process streams. Thus, the discovery of the process of the instant invention is believed to be a decided advance in the art of the purification of N-acetyl aminophenols contaminated with chromogenic impurities.

### SUMMARY OF THE INVENTION

This invention is directed to a process for the removal of chromogenic impurities from N-acetyl aminophenols. Accordingly, the primary object of this invention is to provide a process for the purification of N-acetyl aminophenols contaminated with chromogenic impurities.

An additional object of this invention is to provide a process for the purification of N-acetyl aminophenols readily adaptable to batch, semicontinuous and continuous operations.

These and other objects, aspects, and advantages of the instant invention will become apparent to those skilled in the art from the accompanying description and claims.

The above objects are achieved by the improved process disclosed herein for the purification of N-acetyl aminophenols contaminated with chromogenic impurities where the N-acetyl aminophenol is crystallized from solvent media and the resultant solvent media phase is recycled for use as solvent media for crystallization of additional N-acetyl aminophenol, the improvement comprising treating the solvent media with an ion exchange resin of a type and in a form effective to remove chromogenic impurities or contaminants contained therein prior to recycling for subsequent use.

## DESCRIPTION OF THE PREFERRED EMBODIMENTS

In accordance with this invention, N-acetyl aminophenols contaminated with chromogenic impurities are purified via a process wherein the N-acetyl aminophenol is crystallized from solvent media and the resultant mother liquor or solvent media phase is recycled for use as solvent media for crystallization of additional N-acetyl aminophenol, the improvement comprising treating the solvent media with an ion exchange resin of a type and in a form effective to remove chromogenic impurities or contaminants contained therein prior to recycling for subsequent use. The resultant N-acetyl aminophenol product is essentially free of chromogenic impurities, i.e., color-free, as measured by a limit of color (LOC) test.

The terms "chromogenic impurities" and "chromogenic contaminants" are employed interchangeably herein to mean colored and/or color-forming or color-producing impurities or contaminants.

N-acetyl aminophenols which can be purified in accordance with the process of the instant invention are in general the so-called crude N-acetyl aminophenols which are the direct reaction product of the acetylation of the corresponding aminophenol with acetic anhydride, acetic acid, or mixtures thereof in a suitable reaction vessel and under conditions well known to those skilled in the art. It is to be understood, however, that the process of the instant invention is also suitable for the removal of chromogenic impurities from N-acetyl aminophenols by whatever process produced. In any event, regardless of the method or process of production, suitable N-acetyl aminophenols include those in which the amino group is in a position ortho or para to the hydroxy group (i.e., having an orientation other than meta with respect to each other). Exemplary of such N-acetyl aminophenols are N-acetyl-o-aminophenol, N-acetyl-p-aminophenol (also known as acetaminophen or APAP), N-acetyl-6-aminocarvacrol, N-acetyl-4-aminocarvacrol, N-acetyl-6-aminothymol, N-acetyl-4-aminothymol, N-acetyl-2,5-dimethyl-4-aminophenol, N-acetyl-2-methyl-4-amino-phenol, N-acetyl-3-methyl-4-aminophenol, N-acetyl-2,5-diethyl-4-aminophenol, N-acetyl-3-n-butyl-4-aminophenol, and other N-acetyl alkyl-substituted aminophenols containing lower alkyl nuclear substituents, where the term "lower alkyl", as employed herein, refers to substituents containing 1-4 carbon atoms. Among these N-acetyl aminophenols, N-acetyl-p-aminophenol (APAP) is preferred due to its commercial significance as a pharmaceutical product.

Solvents suitable for use as the solvent media in the process of the instant invention are those in which the N-acetyl aminophenol is readily soluble at elevated temperatures (at or near the boiling point of the solvent) but only sparingly soluble at lower temperatures, such as, for example, 50°C or less.

Such solvents also should be capable of dissolving and retaining in solution the chromogenic impurities at desirable operating temperatures. Nonlimiting representatives of suitable solvents include water and mixtures of alcohols containing 1-3 carbon atoms and water, such as methanol/water, ethanol/water, and 2-propanol/water. A convenient alcohol/water volume ratio in such mixtures ranges from about 20/80 to about 30/70. Of these solvents, water is preferred, particularly for N-acetyl-p-aminophenol (APAP), in that it is readily available and the least expensive of such solvents.

The ion exchange resins employed in the process of the instant invention are the resins of a type and in a form effective to remove the chromogenic impurities from the solvent media (from which the N-acetyl aminophenol has been recrystallized) prior to recycling such solvent media for recrystallizing subsequent batches of N-acetyl aminophenol. Suitable resins include strongly acidic cation exchange resins (which are sulfonated copolymers of styrene and divinylbenzene) in the hydrogen or alkali metal (e.g., sodium) form and anion exchange resins (which are epichlorohydrin-amine condensates and acrylic polymers and styrene-divinylbenzene colpolymers containing primary, secondary, tertiary amine, and quaternary ammonium functionalities, or mixtures thereof) which may range from weakly basic [in the (amine) free base form] to strongly basic [in the (quaternary ammonium) hydroxide form], and mixtures of the cation exchange resins and the anion exchange resins.

In general, preferred ion exchange resins are strongly acidic cation exchange resins in the sodium form and mixtures of a strongly acidic cation exchange resin in the hydrogen from and a strongly basic anion exchange resin (in the quaternary ammonium hydroxide form). When the mixture of strongly acidic cation exchange resin and strongly basic anion exchange resin is employed, the proportion of cation exchange resin to anion exchange resin conveniently will be about 50/50 on a weight basis. The actual proportion of cation exchange resin to anion exchange resin, as well as the overall amount of resin, employed, however, is not narrowly critical and in general will depend upon the amount and type of chromogenic impurities, such as aminophenol and derivatives thereof, and the acidic or basic nature of such impurities.

In operation of the process of the instant invention, the solution of N-acetyl aminophenol dissolved in the solvent media is prepared by heating the mixture of N-acetyl aminophenol and solvent media, and optionally (but preferably), sodium metabisulfite (as an antioxidant) to a temperature from about 70°C to about 100°C, preferably from about 75°C to about 98°C, such that the resultant solution contains from about 15% to about 30% by weight N-acetyl aminophenol and a corresponding remaining balance of solvent media. The thus-formed solution is cooled to a temperature sufficient to cause crystallization of the N-acetyl aminophenol. In general, a suitable temperature will range from about 10°C to about 50°C, preferably from about 20°C to about 40°C. The N-acetyl aminophenol crystals precipitated from the solution are separated from the solvent media phase or mother liquor by centrifugation or filtration, washed with fresh solvent, and dried by heating, conveniently to a temperature of about 40°C to about 65°C, or by any conventional means known to the art.

It will be apparent to those skilled in the art that the solvent media from which the N-acetyl aminophenol is recrystallized remains substantially saturated with N-acetyl aminophenol. As a result, in order to minimize loss of N-acetyl aminophenol, the solvent media is recycled for use in recrystallizing subsequent batches of N-acetyl aminophenol. However, in accordance with the process of the instant invention, due to the presence of chromogenic impurities in the solvent media, which if not removed, serve to contaminate N-acetyl aminophenol recrystallized therefrom, the solvent media is treated with an ion exchange resin of a type and in a form effective to remove the chromogenic impurities prior to recycling the solvent media for subsequent use. In a preferred embodiment, the solvent media is filtered through a bed of the ion exchange resin and recycled for subsequent use.

The N-acetyl aminophenols purified in accordance with the process of the instant invention are essentially free of chromogenic impurities, i.e., they are color-free, as measured by an LOC test and are suitable for use in pharmaceutical compositions and other demanding applications.

The following specific examples illustrating the best presently-known methods of practicing this invention are described in detail in order to facilitate a clear understanding of the invention. It should be understood, however, that the detailed expositions of the application of the invention, while indicating preferred embodiments, are given by way of illustration only and are not to be construed as limiting the invention since various changes and modifications within the spirit of the invention will become apparent to those skilled in the art from this detailed description.

### EXAMPLE 1 (Comparative)

Crude APAP (prepared in accordance with Example 5 of U.S. Patent 4,670,589) was admixed with recycled mother liquor from a previous APAP purification run, demineralized water, and sodium metabisulfite ($Na_2S_2O_5$, 250 ppm, based on the total volume of mixture) to provide an aqueous mixture containing about 16% to about 28% APAP. The mixture was heated to a temperature from about 90°C to about 100°C to form a homogeneous solution and filtered through a bed of activated carbon. The resultant solution was cooled to a temperature of about 15°C to about 50°C to crystallize the APAP. The APAP was collected by centrifugation, washed with fresh demineralized water, and dried at a temperature from about 40°C to about 65°C. The mother liquor was maintained at a temperature from about 30°C to about 50°C to ensure solution homogeneity and gravity filtered through a bed of activated carbon in a vertically oriented column designed to ensure uniform velocity distribution over the column cross section. The filtered mother liquor (30%) was recycled for use in recrystallizing subsequent batches of crude APAP. The procedure was repeated a total of seven times to yield APAP which exhibited an average limit of color (LOC) value of 10.14 x $10^{-3}$.

The LOC value was determined as follows: APAP (50.0 g) was slurried with 50 ml of methanol at room temperature (approximately 25°C) for 3 min. The undissolved APAP was separated from the liquid phase by centrifugation or filtration. The absorbance of the resultant liquid phase was measured at 420 nm to provide the LOC value. The average LOC value was determined by averaging the LOC values from the total number of APAP recrystallization runs.

### EXAMPLE 2

The apparatus and procedure described in Example 1 was employed except that the activated carbon bed employed in Example 1 to filter mother liquor was replaced with an approximately 50:50 weight mixture of strongly acidic cation exchange resin in the hydrogen form and strongly basic anion exchange resin in the hydroxide form (Dow MR-3 resin). The mother liquor was gravity filtered through the bed of ion exchange resin and about 80% of the filtered mother liquor was recycled for use as described in Example 1. The procedure was repeated until APAP in excess of 200.0 kg of APAP per kg of resin (200.0 lb per lb of resin) employed was produced. The APAP produced, except for a brief upset period (approximately 7.6% of the on-stream time) caused by insufficient washing of the crude APAP during collection, exhibited an average LOC value of 7.22 x $10^{-3}$. The average LOC value of the APAP produced during the upset period was 27.16 x $10^{-3}$.

### EXAMPLE 3

The apparatus and procedure described in Example 2 was employed except that the column was packed with strongly acidic cation exchange resin in the sodium form (Dow HCR-W2 resin). The mother liquor was filtered through the bed of ion exchange resin and about 80% of the filtered mother liquor was recycled for use in recrystallizing subsequent batches of crude APAP. The procedure was repeated until APAP in excess of 200.0 kg of APAP per kg of resin employed was produced. The APAP produced, except for a brief upset period (approximately 5.5% of the on-stream time) as described in Example 2, exhibited an average LOC value of 9.13 x $10^{-3}$. The average LOC value for the APAP produced during the upset period was 36.80 x $10^{-3}$.

Thus, it is apparent that there has been provided, in accordance with the instant invention, a process for the purification of N-acetyl aminophenols containing chromogenic impurities that fully satisfies the objects and advantages set forth hereinabove. While the invention has been described with respect to various specific examples and embodiments thereof, it is understood that the invention is not limited thereto and that many alternatives, modifications, and variations will be apparent to those skilled in the art in light of the foregoing description. Accordingly, it is intended to embrace all such alternatives, modifications, and variations.

## Claims

1. A process for the purification of N-acetyl aminophenols containing chromogenic impurities wherein the N-acetylaminophenol is crystallized from a solvent medium by the steps of :
(a) dissolving the N-acetylaminophenol in the solvent medium to form an N-acetylaminophenol-solvent medium solution;
(b) inducing N-acetylaminophenol crystal formation in the N-acetylaminophenol-solvent medium solution to form N-acetylaminophenol crystal phase and a solvent medium phase containing the chromogenic impurities
(c) separating the N-acetylaminophenol crystal phase from the solvent medium phase; and
(d) recycling the solvent medium phase to step (a) for use as the solvent medium;
characterised by removing chromogenic impurities from the solvent medium phase separated in step (c) by treatment with an ion exchange resin prior to recycling in step (d).

2. A process of Claim 1, wherein the ion exchange resin is a strongly acidic cation exchange resin in the alkali metal form.

3. A process of Claim 2, wherein the alkali metal of the strongly acidic cation exchange resin is sodium.

4. A process of Claim 1, wherein the ion exchange resin is a mixture of strongly acidic cation exchange resin in the hydrogen form and a strongly basic anion exchange resin in the hydroxide form.

5. A process improvement of Claim 4, wherein the strongly acidic cation exchange resin/strongly basic anion exchange resin weight ratio is about 50/50.

6. A process of any of the preceding claims, wherein the solvent medium is water.

7. A process of any of Claims 1 to 5, wherein the solvent medium is an alcohol/water mixture.

8. A process of Claim 7, wherein the alcohol of the alcohol/water mixture is methanol, ethanol, or 2-propanol.

9. A process of either Claim 7 or Claim 8, wherein the alcohol/water volume ratio is from 28/80 to 30/70.

10. A process of any of the preceding claims, wherein the N-acetylaminophenol crystal formation is induced by cooling the N-acetylaminophenol-solvent medium solution to a temperature from 10°C to 50°C.

11. A process of Claim 10, wherein the solution is cooled to a temperature from 20°C to 40°C.

12. A process of any of the preceding claims, wherein the N-acetylaminophenol crystal phase is separated from the solvent medium phase by centrifugation.

13. A process of any of the preceding claims, wherein the N-acetylaminophenol is N-acetyl-p-aminophenol.